# EUROPEAN PATENT APPLICATION

(11) **EP 3 260 759 A1**
(43) Date of publication of application: **27.12.2017**
(21) Application number: 16175819.8
(22) Date of filing: 22.06.2016
(51) Int. Cl.: F17C 7/02, F17C 13/08, G01F 22/00, G01F 22/02, A61M 16/06, A61M 16/20, G01F 15/00

(54) **SUPPLY UNIT FOR MEDICAL OXYGEN STORED IN LIQUID FORM AND METHOD OF ESTIMATING THE LEVEL OF LIQUID OXYGEN IN SUCH A SUPPLY UNIT**

(71) Applicant: Linde Aktiengesellschaft, 80331 München (DE)
(72) Inventor: Bause, Matthias, 82205 Gilching (DE); Häußermann, Sabine, 82061 Neuried (DE); Schmehl, Wolfgang, 82031 Grünwald (DE)
(74) Representative: Christie, Gemma Louise

(57) **Abstract**

The invention provides a supply unit (10, 20) for liquid medical oxygen, the supply unit (10, 20) including a reservoir (11, 21) adapted to be filled with the liquid medical oxygen, at least one dispensing valve (13, 19, 23) in fluid communication with the reservoir (11, 21) and operable by a user of the supply unit (10, 20), and an overpressure valve (14, 24) in fluid communication with the reservoir (11, 21) and adapted to open in order to vent the reservoir (11, 21) when a pressure in the reservoir (11, 21) exceeds a given value. The mobile supply unit (10, 20) includes estimating means adapted to provide a value corresponding to an amount of the liquid medical oxygen spent from the reservoir (11, 21) since an initial timepoint at least on the basis of a value corresponding to an initial amount of the liquid medical oxygen in the reservoir (11, 21) at the initial timepoint, a value corresponding to one or more valve settings of the dispensing valve or valves (13, 19, 23) since the initial timepoint, and a value corresponding to one or more durations during which the overpressure valve (14, 24) was open in order to vent the reservoir (11, 21) since the initial timepoint. A corresponding method is also part of the present invention.

## Description

The present invention relates to a supply unit for medical oxygen stored in liquid form and to a method of estimating the level of liquid oxygen in a such a supply unit according to the precharacterising clauses of the independent claims.

### Prior art

Patients with impaired pulmonary function, e.g. individuals suffering from chronic obstructive pulmonary disease (COPD) or from otherwise diseased or damaged lungs, are sometimes severely restricted in their mobility due to dyspnoea. Besides the negative consequences on participation in daily life, immobility may cause muscular wasting and osteoporosis. To avoid such consequences, long term oxygen therapy (LTOT) may be used. In LTOT, which is effective in improving patient mobility but which, when delivered over extended periods, also increases general life expectancy, patients are supplied with supplemental oxygen from an oxygen supply unit. The oxygen supply unit is preferentially mobile and may thus be carried, pushed on wheels or moved together with a walking aid or a wheelchair by the patient receiving LTOT.

In mobile oxygen supply units for oxygen therapy, liquefied oxygen (LOX) or pressurized gaseous oxygen (GOX) may be stored and delivered therefrom, or such mobile oxygen supply units may include a portable oxygen concentrator producing oxygen or a gas enriched in oxygen from ambient air. LOX systems are considered advantageous due to their large range. In contrast to GOX systems or systems with oxygen concentrators which depend on electricity provided by a battery, patients may thus be supplied over extended periods without the need of refilling the mobile oxygen supply unit or recharging the battery of a portable concentrator.

Conventional medical LOX systems include a comparatively large stationary LOX storage tank which is located at and remains in the patient's home. The stationary LOX storage tank is periodically refilled from a mobile LOX storage vessel, typically a lorry tanker. A mobile LOX supply unit is refilled by the patient from the stationary LOX storage tank when necessary. When a patient is at home, LTOT may also be delivered directly from the stationary LOX storage tank. In this case, the stationary LOX storage tank is also a "supply unit" according to the language as used herein.

A corresponding system is e.g. disclosed in US 6,742,517 A. In such a system, the mobile LOX supply unit may e.g. be refilled by firmly forcing a port of the mobile LOX supply unit onto a corresponding port of the stationary LOX storage tank. When the ports, which include suitable valves, are thus brought into engagement, LOX will stream from the LOX storage tank into the mobile oxygen supply unit. The ports may be interlocked with each other and/or further interlocks between the stationary LOX storage tank and the mobile LOX supply unit may be used during filling.

In some systems according to the prior art, the user must manually open a vent valve on the exterior of the mobile LOX supply unit into an open position to allow gas in the mobile LOX supply unit to be replaced by LOX while pressing down the mobile oxygen supply unit in order to keep the ports in engagement. This requires using both hands or using more than one person to fill the mobile LOX supply unit.

In US 8,156,972 B2, a LOX system including a stationary LOX storage tank with a turntable is disclosed. The turntable is rotatable with respect to the stationary LOX storage tank. An interface is provided in or on the turntable. The interface has a shape generally corresponding to a shape of a portion of the mobile LOX supply unit. When the turntable is rotated, the corresponding ports of the stationary LOX storage tank and the mobile LOX supply unit engage and the latter may be filled.

It is desirable for a patient, when using a mobile LOX supply unit, or when LOX is delivered directly from the stationary LOX storage tank, to be able to obtain an indication of how much LOX is spent from and/or how much LOX is left in the mobile LOX supply unit or the stationary LOX storage tank in order to estimate the remaining usage time until a refill is necessary. Alternatively, it could be desirable for a patient to be able to obtain a direct indication of the remaining usage time until such a refill.

However, determining the amount of LOX left in a mobile LOX supply unit, but also in a stationary LOX storage tank, may be problematic. In contrast to systems based on pressurized GOX wherein an initial pressure after refilling may be compared with the actual pressure at a given timepoint, this is not possible in LOX systems because LOX is stored at atmospheric pressure or only slightly above. For example it is known to determine the weight of a mobile LOX supply unit with a sensor included in a shoulder carrying strap, but due to the permanent motion of the mobile oxygen supply unit, the weight determination may be imprecise. Also level sensors, especially in mobile LOX supply units, are often unreliable due to motion.

Consequently, there is a need for improved means of determining a value corresponding to an amount of LOX remaining in, or spent from, a supply unit for medical oxygen stored in liquid form.

### Summary of the invention

According to the present invention, a supply unit for medical oxygen stored in liquid form and to a method of estimating the liquid oxygen level in such a supply unit with the features of the independent claims is provided. Preferred embodiments are subject of the dependent claims and of the description that follows.

### Detailed description of the invention

The present invention is based on the finding that an amount of LOX remaining in, or spent from, a supply unit for medical oxygen as described above, i.e. either a mobile LOX supply unit or a stationary LOX storage tank, can be determined or estimated on the basis of a valve setting of at least one oxygen dispensing valve and an initial amount of oxygen in a reservoir of the supply unit, if an amount of oxygen blown off via an overpressure valve is also considered. In contrast to the rather unreliable methods as used in the prior art, the present invention allows for a more precise prediction of how much LOX is left and how long oxygen therapy may still be administered. According to preferred embodiments of the invention, "intelligent" oxygen supply systems on the basis of LOX are provided that allow for an individualized treatment of specific patients or patient groups according to their oxygen demand on the basis of sensed patient parameters.

The present invention provides a supply unit for liquid medical oxygen which may be, as mentioned, either a mobile LOX supply unit or a stationary LOX storage tank. The supply unit includes a reservoir adapted to be filled with the liquid medical oxygen. Such a reservoir is preferentially thermally isolated, e.g. via a vacuum insulation, as generally known from mobile supply units of the prior art. The supply unit further comprises at least one dispensing valve in fluid communication with the reservoir and operable by a user of the mobile supply unit.

If the supply unit is a mobile LOX supply unit as described above, typically only one dispensing valve is present which is preferentially in fluid communication with a patient oxygen delivery unit, e.g. a nasal cannula. The oxygen delivery unit may be attached via a suitable coupling and with corresponding tubing to a port of the oxygen delivery unit which is in fluid communication with the dispensing valve.

If a stationary LOX storage tank is used as the supply unit, oxygen therapy may be administered directly from the stationary LOX storage tank, as mentioned. In this case, a dispensing valve for this purpose is present which is, comparable to the dispensing valve described for the mobile LOX supply unit above, preferentially in fluid communication with a patient oxygen delivery unit. Furthermore, however, a dispensing valve is present in this case which is used for refilling one or more mobile LOX supply units as described above. In this case, oxygen withdrawn from the reservoir via both the dispensing valves reduces the remaining oxygen level and thus needs to be considered when calculating the remaining usage time.

The supply unit further includes an overpressure valve in fluid communication with the reservoir and adapted to open in order to vent the reservoir when a pressure in the reservoir exceeds a given value. As LOX is preferentially stored at atmospheric pressure or slightly above in a stationary LOX storage tank as initially described and also in the reservoir of a mobile LOX supply unit, the given value for the pressure in the reservoir may e.g. be 1,1 bar, 1,2 or 1,5 bar (abs.). If the pressure in the reservoir exceeds this given value, the overpressure valve opens and releases a part of the gas from the reservoir into the atmosphere. A pressure increase in the reservoir is e.g. possible if the supply unit is exposed to increased temperatures or if, over longer periods of time, little or no oxygen is withdrawn via the oxygen dispensing valve(s). Evaporating LOX, in such cases, raises the pressure in the reservoir.

According to the present invention, the supply unit includes estimating means adapted to provide a value corresponding to an amount of the liquid medical oxygen spent from the reservoir since an initial timepoint on the basis of a value corresponding to an initial amount of the liquid medical oxygen in the reservoir at the initial timepoint, a value corresponding to one or more valve settings of the dispensing valve or valves since the initial timepoint, and a value corresponding to one or more durations during which the overpressure valve was open in order to vent the reservoir since the initial timepoint. The initial timepoint may be a timepoint at which the amount of oxygen in the reservoir is known exactly, e.g. via a measurement or after a complete refill of the reservoir. A complete refill can, e.g., be determined via a floating level indicator and/or via a blow off of excessive LOX after a complete refill. Also an exact measurement of the weight of a mobile LOX supply unit is possible, e.g. with a sufficiently precise balance of a stationary LOX storage tank during refill.

The value corresponding to one or more valve settings of the dispensing valve or valves since the initial timepoint which is used according to the present invention may be the result of an observation of the valve setting or settings over time, and may be provided on the basis of a signal of a valve position sensor. Knowing the valve setting or settings over time, the amount of oxygen dispensed via the dispensing valve or valves may be determined, and thus the amount of oxygen spent via this valve or these valves. Such a value may also be an accumulated value of oxygen spent via the dispensing valve or valves since the initial timepoint.

The value corresponding to one or more durations during which the overpressure valve was open in order to vent the reservoir since the initial timepoint may be a cumulated time the overpressure valve was open or a value corresponding thereto. Including such a value in the determination or estimation of the value corresponding to an amount of the liquid medical oxygen spent from the reservoir since an initial timepoint increases precision and makes sure that the amount of LOX in the reservoir is not overestimated, even if the supply unit is e.g. exposed to high temperatures or a comparatively large proportion has been lost by evaporation during phases of non use.

While especially existing mobile LOX supply units are passive dewars whose only function is to store liquid oxygen and to evaporate the amount of oxygen needed by the patient, and which do not allow for an indication of remaining time of therapy for a patient using the mobile supply unit, and while existing filling status displays of the stationary LOX storage tanks give only a rough estimate of the remaining amount of oxygen, the present invention allows using "intelligent" technologies that calculate the remaining time for oxygen therapy and, according to preferred embodiments of the present invention as outlined below, allow for a patient-specific oxygen dosage. Such technology was previously only available for cylinders filled with GOX but not for LOX systems due to different physico-chemical properties, the operation mode and technical demands of a LOX vessel as compared with a gas cylinder. From the values mentioned, i.e. the value corresponding to one or more valve settings of the dispensing valve or valves and the value corresponding to one or more durations during which the overpressure valve was open, which represent a "history" of LOX usage, an extrapolation into the future is possible with some certainty and thus patient safety and comfort can be improved.

Oxygen consumption and related remaining duration of therapy are variable and depend on the activities of the patient activities, flow settings, and an eventual activation of overpressure valves. In conventional mobile LOX supply units, patients therefore often have a feeling of insecurity when being away from home. This even intensifies as, in conventional mobile LOX supply units, typically there is no chance to react in situations when the LOX is about to be spent. In the worst case, the patient is away from home with the mobile LOX supply unit and the reservoir runs out of oxygen. In such a case, in conventional mobile LOX supply units, it is up to the patient to organise help or set off an emergency call. This might be a risky situation as patients usually are elderly and tend to panic in such situations. The present invention, in contrast, allows for an early warning preventing such situations or in an automatic reaction, e.g. an automatic emergency call, should LOX be spent.

A mobile LOX supply unit according to the present invention may be equipped with data receiving, storage, handling, and transmission modules which by means of algorithms are able to calculate the remaining time of oxygen therapy for the patient. A GPS sensor for continuous tracking the actual location of the patient may also be used, allowing for locating the patient in case of an emergency. The mobile LOX supply unit according to the present invention may also be equipped with means for displaying relevant information on supply status, the remaining time of therapy or others to the patient while using the mobile LOX supply unit or relevant information regarding the refilling process when and while the mobile LOX supply unit is filled from the stationary LOX storage tank.

A mobile LOX supply unit or a stationary LOX reservoir according to the present invention may, according to a particularly preferred embodiment, also comprise means that are adapted to measure or determine at least one further patient parameter like movement, breathing rate, heart rate, blood pressure, skin temperature or body temperature. As mentioned below, particularly a stationary LOX reservoir may act as a data hub to which such additional patient data may be transmitted for evaluation, storage and/or further transmission to a clinician for diagnosis. Individualized treatment of the specific patient thus becomes possible. Due to the continuous monitoring by the respective LOX supply unit, to which the patient is permanently connected to, diagnostic data become more reliable as compared to measurements via separate devices, which may be forgotten by the patient and generally rather represent possibly unrepresentative random samples. In the stationary LOX reservoir and/or in the mobile LOX supply unit, supplementary parameters may be entered by the patient or by a clinician, like age, sex, body weight, activity status, etc. These supplementary parameters can also be remotely transmitted to and between the respective units.

For example, by taking into account an activity status (the patient may e.g. walk around, sit, or may be asleep), different dosage levels of oxygen may be appropriate. In some cases, e.g. to increase physical ability for a limited period of time, e.g. if the patient intends to climb stairs, the oxygen dosage level may be increased above a regular level which is intended to avoid hypercapnic respiratory failure in LTOT. In other words, an increase in carbon dioxide during a short time may be accepted while in a long term view oxygen dosage is limited to a level avoiding such a carbon dioxide increase. Phases of increased or decreased physical activity may e.g. be determined by registering an increased heart rate and/or an increased breathing rate via the mobile LOX supply unit or sensors attached thereto.

To differentiate phases of increased activity from other possible causes of an increased heart rate and/or an increased breathing rate like infection and inflammation, a change rate of the heart rate and/or the breathing rate can be evaluated. In phases of increased activity, generally, the heart rate and the breathing rate rises comparatively fast while in infection and inflammation, the increase is rather slow. Increased physical activity may also be determined by a motion sensor which is part of the mobile LOX supply unit. By observing the heart rate and/or the breathing rate, especially in connection with the detection of motion, the system according to the present invention may advantageously also be used as an activity sensor. For example, different activity levels for a specific patient may also be defined, based on the heart rate and/or the breathing rate. By further evaluating the supplementary parameters mentioned above, the patient may be provided with a highly individualized treatment. This is especially of high relevance for some groups of patients, as outlined in the following.

Excessive oxygen administration may lead to hypercapnic respiratory failure in some chronic obstructive pulmonary disease (COPD) patients. This is caused, as presently understood, by a ventilation-perfusion (Va/Q) mismatch and by the Haldane effect. For details, reference is made to the literature, e.g. W.F. Abdo and L.M.A. Heus, "Oxygen-induced hypercapnia in COPD: myths and facts", Crit. Care 2012, 16(5), 323, and D. Lynes, "Managing hypoxia and hypercapnia", Nurs. Times 2003, 99(11), 57.

For most COPD patients, a saturation of arterial oxygen (Sa02) of 88 to 92%, compared with 94 to 98% for patients not at risk of hypercapnic respiratory failure, should be aimed at. However, considering values like SaO2 or the partial pressure of arterial oxygen (PaO2) alone may not be sufficient for long-duration oxygen treatment which is indicated in some COPD patients to avoid severe consequences of hypoxia. Over time, carbon dioxide elimination in such patients can vary, allowing for a higher or lower oxygen dosage to be tolerated without the risk of hypercapnic respiratory failure. Obviously, as long as hypercapnic respiratory failure can be avoided, a higher oxygen dosage is beneficial for COPD patients, e.g. in view of a greater physical ability.

According to a preferred embodiment of the present invention, a supply unit for medical oxygen is thus additionally adapted to regulate the amount of oxygen provided to the patient on the basis of an oxygen status and of the basis of a carbon dioxide status of the patient. An "oxygen status" or a "carbon dioxide status" may, in this context, refer to any value describing, indicating, or being related to, a level or concentration of oxygen or of carbon dioxide in the blood of the patient, especially an arterial saturation value (SaO2, SaCO2) or an arterial partial pressure (PaO2, PaCO2). The oxygen status and the carbon dioxide are advantageously determined via a non-invasive transcutaneous measurement, but can be provided in any way suitable and known from the prior art. According to a corresponding embodiment of the present invention, suitable sensing means are provided at, or connected to, the mobile LOX supply unit or the stationary LOX reservoir.
The modules of the oxygen supply system that is formed according to the present invention, especially mobile LOX supply devices and the stationary LOX reservoir, are preferably able to remotely and wireless receive external data from computers, tablets, smartphones etc. which provide patient parameters and supplementary parameters as mentioned above, and additionally or alternatively target values for oxygen saturation to allow for a titrated oxygen supply. The modules are preferably adapted to receive data from additional or integrated oxygen saver devices (so-called demand systems). Via the combined informations from sensors and external data, the modules are able to steer oxygen flow in a way that allows target specific oxygen supply.

A number of warnings may, according to preferred embodiments of the present invention, be issued locally and/or remotely, in order to increase patient safety. For examples, the individual modules as mentioned above may be adapted to recognise the risk of a developing hypercapnia and to automatically prevent oxygen overdosing beyond critical limits. The modules may also be adapted to create alarms/warnings if the oxygen supply status and/or the physiologic status of the patient as determined by the sensors described above are running out of preset limits. Such alarms/warnings may also be transmitted to a physician, hospital, emergency call center etc., e.g. in case of a critical patient physiologic status (e.g. the oxygen status falls under, or the carbon dioxide status raises beyond, a certain, predefined alarm limit). Additionally, the modules may be provided with means adapted to automatically or on demand (e.g. by a simple push button or soft key at a display) create an emergency call at the patient's service provider. Thus, a patient may request an emergency intervention by the service provider in case of health problems.

Refilling of stationary LOX storage tanks is conventionally based on a fixed schedule. The real need of refill can be, as for the mobile LOX supply units mentioned above, variably depend on the activities of the patient, handling errors during the process of refilling mobile LOX supply units and the activation of the overpressure valve. A refill service based on a routine fixed delivery schedule may thus be independent from real needs. This can lead to suboptimal supply situations wherein either an oversupply with health economic issues and high costs for the LOX provider or an undersupply, with the consequences mentioned above, may occur.

The present invention, in contrast, may allow for an automated order at the service provider who is contracted to refill the stationary LOX storage tank. This may be performed by any kind of data connection between service provider and the stationary LOX storage tank. Based on the values obtained in the context of the present invention, the need for maintenance or exchange of the stationary LOX storage tank can be detected, and respective orders can be sent automatically to the service provider as well, e.g. if an unusually high overpressure valve opening rate indicates lacking insulation and thus excessive evaporation.

Data storage, handling and transmission modules can also be used to act as data hub for communication of patient specific data as well as compliance data to the healthcare professionals in charge of the patient. The intelligent features enable the stationary vessel to be a data hub for any kind of telehealth applications for the patient such as, but not limited to, monitoring of patient adherence to long term oxygen therapy. In this context, data measured via the sensors of the stationary LOX storage tank or the mobile LOX vessel as mentioned above may also be evaluated.

According to a preferred embodiment of the present invention, the supply unit further includes a pressure sensor adapted to provide a pressure value corresponding to a pressure in the reservoir, wherein the estimating means are adapted to determine the value corresponding to the duration during which the overpressure valve was open in order to vent the reservoir since the initial timepoint on the basis of the pressure signal provided by the pressure sensor. This allows for an indirect determination at which times the overpressure valve was open via the pressure in the reservoir. The overpressure valve itself, in contrast, can be provided as a fully mechanical valve which is independent from electrical energy and thus provides additional safety.

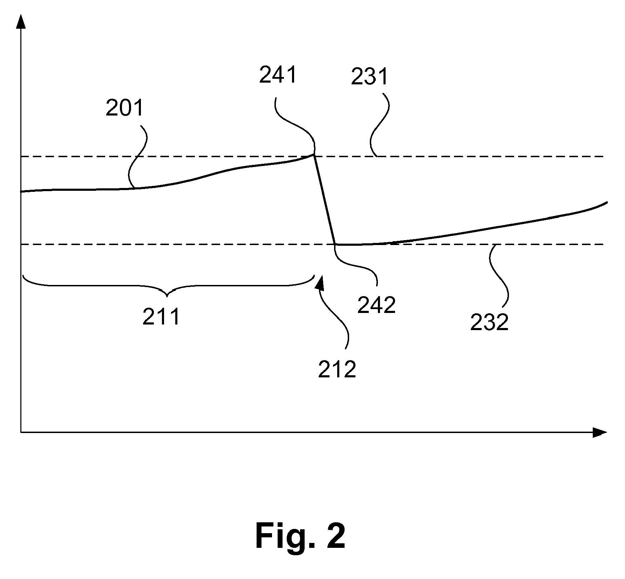

In the supply unit, according to a preferred embodiment of the present invention, the estimating means are adapted to determine the value corresponding to the duration during which the overpressure valve was open in order to vent the reservoir since the initial timepoint on the basis of the signal of the pressure sensor by determining timepoints of opening and closure of the overpressure valve in the pressure signal provided by the pressure sensor. As also explained with reference to Figure 2, a pressure signal over time shows a characteristic shape when the overpressure valve repeatedly opens. From characteristics of such a signal, thus, the times the overpressure valve was open may be determined.

Especially, according to a preferred embodiment of the present invention, the timepoints of opening and closure of the overpressure valve in the pressure signal provided by the pressure sensor are local extrema in the pressure signal or a signal derived from the pressure signal, e.g. a filtered pressure signal over time. A maximum is typically present immediately before the overpressure valve opens and a minimum is present immediately before the overpressure valve closes.

Alternatively to the determination via the pressure in the reservoir, the times the overpressure valve opened may also be determined on the basis of a flow rate of gas through the overpressure valve. The supply unit according to a preferred embodiment of the present invention, therefore, further includes a gas flow sensor adapted to provide a gas flow value corresponding to a gas flow through the overpressure valve, wherein the estimating means are adapted to determine the value corresponding to the duration during which the overpressure valve was open in order to vent the reservoir since the initial timepoint on the basis of the gas flow value provided by the gas flow sensor. In this case, an accumulated gas flow may easily be determined and the lost LOX may be directly measured.

A mobile supply unit according to the present invention may, however, also include a valve state sensor adapted to provide a valve state value corresponding to a valve state of the overpressure valve, wherein the estimating means are adapted to determine the value corresponding to the duration during which the overpressure valve was open in order to vent the reservoir since the initial timepoint on the basis of the valve state value provided by the valve state sensor. In this case, if the hysteresis behaviour of the overpressure valve is known, i.e. the pressure values at which the valve opens and then closes again, and if the amount of evaporated LOX streaming through the valve at these pressures and therebetween is known, one may easily obtain the amount of evaporated LOX blown off during an opening period.

In any case, according to a preferred embodiment of the present invention, in the supply unit the estimating means are adapted to consider at least one valve characteristic of the overpressure valve when providing the value corresponding to the amount of the liquid medical oxygen spent from the reservoir since the initial timepoint. The at least one valve characteristic of the overpressure valve may include at least one of a hysteresis characteristic, a dependency of pressure and flow rate and a valve reaction time. If such characteristics are considered, an amount of evaporated LOX blown off during an opening period, as described in an example above, may be determined with considerable reliability.

To set the initial timepoint taken as the basis for the estimation according to the present invention, reset means operable by a user of the mobile supply unit may be provided according to a preferred embodiment of the invention. In this case, the estimating means are adapted to reset the initial timepoint to an actual timepoint when the user operates the reset means. For example, the user may operate the reset means just after refilling the supply unit.

Alternatively or additionally, according to a preferred embodiment of the invention, the estimating means may be adapted to determine a refill timpepoint at which the reservoir is refilled from an external source of liquid medical oxygen, and to reset the initial timepoint to the refill timepoint upon such a determination. The refill timpepoint may e.g. determined upon the finding that the reservoir is completely filled. As any kind of user interaction bears the risk of human error, expecially in the elderly population in need for LTOT, such an automatic reset may provide advantages over a manual reset as mentioned above.

The present invention also relates to a method of estimating a level of liquid medical oxygen in a supply unit for medical oxygen stored in liquid form, the supply unit including a reservoir adapted to be filled with the liquid medical oxygen, at least one a dispensing valve in fluid communication with the reservoir and being operable by a user of the mobile supply unit, and an overpressure valve in fluid communication with the reservoir and adapted to open in order to vent the reservoir when a pressure in the reservoir exceeds a given value. According to the present invention, via estimating means, a value corresponding to an amount of the liquid medical oxygen spent from the reservoir since an initial timepoint is provided, on the basis of a value corresponding to an initial amount of the liquid medical oxygen in the reservoir at the initial timepoint, a value corresponding to one or more valve settings of the dispensing valve or valves since the initial timepoint, and a value corresponding to one or more durations during which the overpressure valve was open in order to vent the reservoir since the initial timepoint.

Preferentially, the method is used in a mobile supply unit as extensively described above. As to features and advantages of such a method, thus, reference is made to these explanations.

### Brief description of the drawings

Figure 1 schematically illustrates a supply system for liquid medical oxygen with oxygen supply units according to an embodiment of the present invention.
Figure 2 shows a pressure in a LOX reservoir in an oxygen supply unit according to an embodiment of the present invention.

### Embodiments of the invention

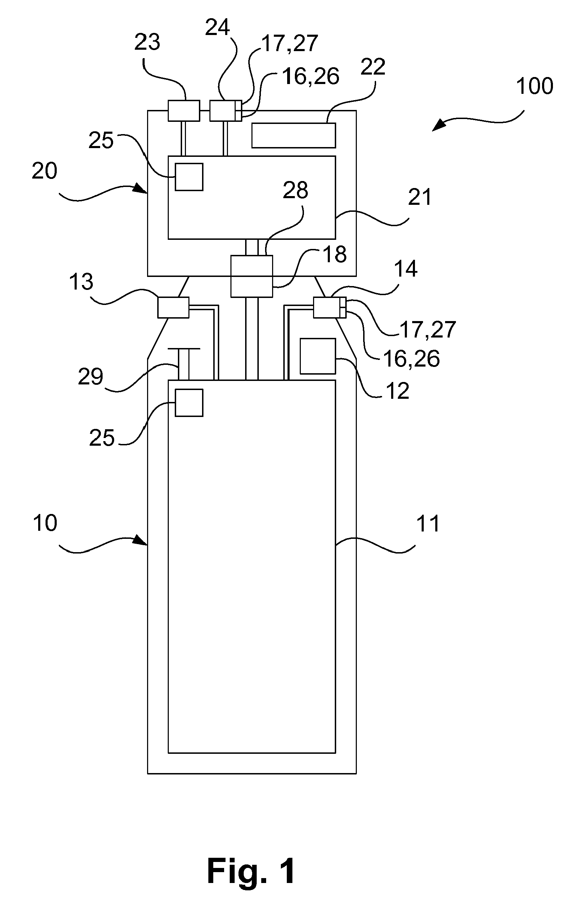

In Figure 1, a supply system for liquid medical oxygen is schematically illustrated and designated 100. The system 100 comprises two supply units 10, 20 for liquid medical oxygen, the supply unit 10 being a stationary LOX storage tank and the supply unit 20 being a mobile LOX supply unit. For ease of reference, elements with basically identical functionality are indicated with reference numerals incremented with 10 for the supply units 10 and 20. These elements are not repeatedly described.

While, with regard to Figure 1, the invention is described with reference to two supply units 10, 20 for liquid medical oxygen, it is to be understood that the present invention can also be used with one supply unit 10, 20 only, i.e. with either a stationary LOX storage tank or a mobile LOX supply unit.

The supply units 10, 20 each include a reservoir 11, 21 adapted to be filled with the liquid medical oxygen. In case of the supply unit 10, i.e. the stationary LOX storage tank, a filling port 29 is provided and adapted to be filled e.g. from a lorry tanker. The supply units 10, 20 each include dispensing valves 13, 23 in fluid communication with the reservoir 11, 21 and operable by a user of the supply unit 10, 20. The dispensing valves 13, 23 may be coupled with an oxygen delivery unit, as mentioned. In case of the supply unit 10, i.e. stationary LOX storage tank, a further dispensing valve 18 is present and adapted to dispense LOX from the reservoir 11 of the stationary LOX storage tank to a reservoir of the supply unit 20 embodied as the mobile LOX supply unit when coupled thereto. The supply unit 20 embodied as the mobile LOX supply unit comprises a corresponding inlet valve 28. Coupling means and ports adapted to mate between the supply units 10, 20 are not shown. The supply units 10, 20 which are shown here in a coupled state may obviously be decoupled to use the supply unit 20 as a mobile LOX supply unit

The supply units 10, 20 each include an overpressure valve 14, 24 in fluid communication with the respective reservoir 11, 21 and adapted to open in order to vent the reservoir 11, 21 when a pressure in the respective reservoir 11, 21 exceeds a given value, as explained above.

In the supply units 10, 20, means are provided, e.g. as a part of control units 12, 22, which are adapted to provide a value corresponding to an amount of the liquid medical oxygen spent from the respective reservoir 11, 21 since an initial timepoint on the basis of a value corresponding to an initial amount of the liquid medical oxygen in the respective reservoir 11, 21 at the initial timepoint, a value corresponding to one or more valve settings of the respective dispensing valve or valves 13, 19, 23 since the initial timepoint, and a value corresponding to one or more durations during which the respective overpressure valve 14, 24 was open in order to vent the respective reservoir 11,21 since the initial timepoint.

In the embodiment shown, the supply units 10, 20 are equipped with pressure sensors 15, 25, respectively, which are adapted to provide a pressure value corresponding to a pressure in the respective reservoir 11, 21. The estimating means are, as explained above, adapted to determine the value corresponding to the duration during which the respective overpressure valve 14, 24 was open in order to vent the respective reservoir 11, 21 since the initial timepoint on the basis of the pressure signal provided by the respective pressure sensor 15, 25.

Alternatively or additionally, gas flow sensors 16, 26 and/or valve state sensors 17, 27 may be provided which are adapted to provide a gas flow value corresponding to a gas flow through the respective overpressure valve 14, 24 and/or a valve state value corresponding to a valve state of the overpressure valve 14, 24. For further details, reference is made to the explanations above.

In Figure 2, a pressure in a LOX reservoir like the reservoir 11 or 21 as shown in Figure 1 is shown as a pressure value 201 on the ordinate over time on the abscissa.

It shall be assumed that, from the LOX reservoir, no LOX is withdrawn in liquid or evaporated form via a dispensing valve as, e.g., one of the dispensing valves 13, 19, 23 as shown in Figure 1.

Therefore, due to continuous LOX evaporation, the pressure value 201 raises in a time period 211 until it reaches a given value, indicated with 231. At the indicated value 231, an overpressure valve like the overpressure valves 14, 24 as shown in Figure 1 opens. Correspondingly, the pressure value 201 rapidly drops during a time period 212 until it reaches a further value, indicated with 232. The difference between the values 231 and 232 describes the hysteresis behaviour of the overpressure valve. The process continues with repeated opening and closing of the overpressure valve. The timepoints at which the overpressure valve opens, indicated with 241, and closes, 242, can be determined as local maxima and minima of the pressure value 201.

## Claims

**1.** A supply unit (10, 20) for medical oxygen stored in liquid form, the supply unit (10, 20) including a reservoir (11, 21) adapted to be filled with the medical oxygen, at least one dispensing valve (13, 19, 23) in fluid communication with the reservoir (11, 21) and operable by a user of the supply unit (10, 20), and an overpressure valve (14, 24) in fluid communication with the reservoir (11, 21) and adapted to open in order to vent the reservoir (11, 21) when a pressure in the reservoir (11, 21) exceeds a given value, **characterized in that** the supply unit (10, 20) includes estimating means adapted to provide a value corresponding to an amount of the liquid medical oxygen spent from the reservoir (11, 21) since an initial timepoint at least on the basis of a value corresponding to an initial amount of the liquid medical oxygen in the reservoir (11, 21) at the initial timepoint, a value corresponding to one or more valve settings of the dispensing valve or valves (13, 19, 23) since the initial timepoint, and a value corresponding to one or more durations during which the overpressure valve (14, 24) was open in order to vent the reservoir (11,21) since the initial timepoint.

**2.** The supply unit (10, 20) according to claim 1, further including a pressure sensor (15, 25) adapted to provide a pressure value corresponding to a pressure in the reservoir (11, 21), wherein the estimating means are adapted to determine the value corresponding to the duration during which the overpressure valve (14, 24) was open in order to vent the reservoir (11, 21) since the initial timepoint on the basis of the pressure signal provided by the pressure sensor (15, 25).

**3.** The supply unit (10, 20) according to claim 2, wherein the estimating means are adapted to determine the value corresponding to the duration during which the overpressure valve (14, 24) was open in order to vent the reservoir (11, 21) since the initial timepoint on the basis of the signal of the pressure sensor (15, 25) by determining timepoints of opening and closure of the overpressure valve (14, 24) in the pressure signal provided by the pressure sensor (15, 25).

**4.** The supply unit (10, 20) according to claim 3, wherein the timepoints of opening and closure of the overpressure valve (14, 20) in the pressure signal provided by the pressure sensor (15, 25) are local extrema in the pressure signal or a signal derived from the pressure signal.

**5.** The supply unit (10, 20) according to claim 1, further including a gas flow sensor (16, 26) adapted to provide a gas flow value corresponding to a gas flow through the overpressure valve (14, 24), wherein the estimating means are adapted to determine the value corresponding to the duration during which the overpressure valve (14, 24) was open in order to vent the reservoir (11, 21) since the initial timepoint on the basis of the gas flow value provided by the gas flow sensor (16, 26).

**6.** The supply unit (10, 20) according to claim 1, further including a valve state sensor (17, 27) adapted to provide a valve state value corresponding to a valve state of the overpressure valve (14, 24), wherein the estimating means are adapted to determine the value corresponding to the duration during which the overpressure valve (14, 24) was open in order to vent the reservoir (11, 21) since the initial timepoint on the basis of the valve state value provided by the valve state sensor (17, 27).

**7.** The supply unit (10, 20) according to any one of the preceding claims, wherein the estimating means are adapted to consider at least one valve characteristic of the overpressure valve (14, 24) when providing the value corresponding to the amount of the liquid medical oxygen spent from the reservoir (11, 21) since the initial timepoint.

**8.** The supply unit (10, 20) according to claim 7, wherein the at least one valve characteristic of the overpressure valve (14, 24) includes at least one of a hysteresis characteristic, a dependency of pressure and flow rate and a valve reaction time.

**10.** The supply unit (10, 20) according to any one of the preceding claims, wherein the estimating means are adapted to determine a refill timpepoint at which the reservoir (11, 21) is refilled from an external source of liquid medical oxygen, and to reset the initial timepoint to the refill timepoint upon such a determination.

**11.** The supply unit (10, 20) according to any one of the preceding claims, further comprising means adapted to measure or determine at least one further patient parameter selected from movement, breathing rate, heart rate, blood pressure, skin temperature and body temperature.

**12.** The supply unit (10, 20) according to any one of the preceding claims, further comprising means adapted to evaluate at least one supplementary parameter selected from age, sex, body weight and activity status.

**13.** The supply unit (10, 20) according to any one of the preceding claims, further comprising means adapted to determine an oxygen status and optionally a carbon dioxide status of the patient, and further adapted to regulate an oxygen amount provided to the patient at least on the basis of the oxygen status and optionally the carbon dioxide status of the patient.

**14.** A method of estimating a level of liquid medical oxygen in a supply unit (10, 20) for liquid medical oxygen, the supply unit (10, 20) including a reservoir (11, 21) adapted to be filled with the liquid medical oxygen, at least one dispensing valve (13, 19, 23) in fluid communication with the reservoir (11, 21) and being operable by a user of the mobile supply unit (10, 20), and an overpressure valve (14, 24) in fluid communication with the reservoir (11, 21) and adapted to open in order to vent the reservoir (11, 21) when a pressure in the reservoir (11, 21) exceeds a given value, **characterized in that**, via estimating means, a value corresponding to an amount of the liquid medical oxygen spent from the reservoir (11, 21) since an initial timepoint is provided on the basis of a value corresponding to an initial amount of the liquid medical oxygen in the reservoir (11) at the initial timepoint, a value corresponding to one or more valve settings of the dispensing valve or valves (13, 19, 23) since the initial timepoint, and a value corresponding to one or more durations during which the overpressure valve (14, 24) was open in order to vent the reservoir (11, 21) since the initial timepoint.

**15.** A method according to claim 14, wherein a mobile supply unit (10, 20) according to any one of claims 1 to 10 is provided.
